# EUROPEAN PATENT APPLICATION

(11) **EP 1 679 071 A1**
(43) Date of publication of application: **12.07.2006**
(21) Application number: 04792003.8
(22) Date of filing: 04.10.2004
(51) Int. Cl.: A61K 31/375, A61K 31/19, A61K 8/99, A61K 8/18, A61P 17/16, A61P 43/00, A23L 1/302, A23K 1/16

(54) **COLLAGEN PRODUCTIN ENHANCER AND PRODUCTION PROCESS AND USE THEREOF**

(30) Priority: 07.10.2003 JP 2003348705
(71) Applicant: KABUSHIKI KAISHA HAYASHIBARA SEIBUTSU KAGAKU KENKYUJO, Okayama-shi, Okayama 700-0907 (JP)
(72) Inventor: MIYATA,S. KK Hayashibara Seibutsu Kagaku Kenkyujo, Okayama-shi, Okayama 7000907 (JP); OKAMOTO,I. KK Hayashibara Seibutsu Kagaku Kenkyujo, Okayama-shi, Okayama 7000907 (JP); USHIO,S. KK Hayashibara Seibutsu Kagaku Kenkyujo, Okayama-shi, Okayama 7000907 (JP); KURIMOTO,M KK Hayashibara Seibutsu Kagaku Kenkyujo, Okayama-shi, Okayama 7000907 (JP)
(74) Representative: Daniels, Jeffrey Nicholas
(86) International application number: PCT/JP2004/014591
(87) International publication number: WO 2005/034938

(57) **Abstract**

The present invention has an object to provide an agent for enhancing collagen production and use thereof, and solves the object by establishing an agent for enhancing collagen production, which comprises as effective ingredients L-ascorbic acid or its derivative and a fatty acid or its derivative, and use thereof.

## Description

### Technical Field

The present invention relates to an agent for enhancing collagen production, more particularly, to an agent for enhancing collagen production, which comprises as effective ingredients L-ascorbic acid and/or its derivative (hereinafter called "L-ascorbic acids", unless specified otherwise) and a fatty acid or its derivative, as well as to preparation and use thereof.

### Background Art

In Japanese Patent Kokai No. 2002-201,883, the applicant of the present invention disclosed an agent for enhancing collagen production, as an agent for enhancing collagen production *in vivo,* which comprises as effective ingredients L-ascorbic acid or its derivative and royal jellies. With such royal jellies, the above agent enhances the ability of enhancing the collagen production by L-ascorbic acids.

The action of enhancing collagen production, exerted by the above agent, tends to increase as the increase of the composition ratio of royal jellies against L-ascorbic acids, however, there has been a drawback that the more one uses costly royal jellies the more the resulting agent for enhancing collagen production becomes expensive. The quality control of the above-identified agent is difficult as a drawback because the action of enhancing collagen production exerted by such agent varies distinctively depending on the origin and the preparation of royal jellies to be incorporated.

Under these conditions, there has been in a great demand the establishment of an agent for enhancing collagen production, which can surely and stably improve the action of enhancing the collagen production by L-ascorbic acids and which can be provided on an industrial scale at a relatively low cost.

### Disclosure of the Invention

The present invention, which was made in view of the prior art, is to provide a novel means for surely and stably improving the action of enhancing the collagen production by L-ascorbic acids.

To solve the above object, the present inventors energetically continued studying to reveal what the specific ingredients contained in raw royal jellies are, postulating that such specific ingredients would enhance the ability of inducing the collagen production by L-ascorbic acids. As a result, the present inventors finally revealed that 10-hydroxy-2-decenoic acid (hereinafter called "10-HDA") present in raw royal jellies is one of the above specific ingredients. They also found that the combination use of 10-HDA and L-ascorbic acids in a prescribed ratio will surely and stably enhance the action of enhancing the collagen production by L-ascorbic acids. Further, they found that, similarly as L-ascorbic acid, even in the case of applying any of L-ascorbic acids other than L-ascorbic acid in combination with any of fatty acids other than 10-HDA to mammals including humans, the desired effect will be exerted. In addition, the present inventors found that the combination use of L-ascorbic acids and fatty acids also exerts the action of enhancing TGF-production *in vivo*.

The present inventors established an agent for enhancing collagen production, which comprises L-ascorbic acids and fatty acids as effective ingredients, and preparation and use thereof; and thus they accomplished this invention.

### Brief Explanation of Accompanying Drawings

[FIG. 1] FIG. 1 is a figure illustrating the level of collagen production when NHDF cells are cultured in the presence or the absence of AA2G (L-ascorbic acid 2-glucoside) and/or 10-HDA.
[FIG. 2] FIG. 2 is a figure illustrating the level of collagen production when NHDF cells are cultured in the presence of AA2G (L-ascorbic acid 2-glucoside) and at different concentrations of 10-HDA.
[FIG. 3] FIG. 3 is a figure illustrating the level of collagen production when NHDF cells are cultured in the presence of AA2G (L-ascorbic acid 2-glucoside) and any one of 10-hydroxy-2-decenoic acid (10-HDA), 10-hydroxydecanoic acid (10H decanoic acid), decanoic acid, 2-decenoic acid, and sebacic acid.
[FIG. 4] FIG. 4 is a figure illustrating the level of collagen production when NHDF cells are cultured in the presence of 10-HDA and L-ascorbic acid or AA2G (L-ascorbic acid 2-glucoside).

### Best Mode for Carrying out the Invention

The agent for enhancing collagen production according to the present invention is a composition capable of surely and stably enhancing the collagen production in living bodies' skin.

The term "L-ascorbic acids" as referred to as in the present invention means L-ascorbic acid and derivatives thereof including salts of L-ascorbic acid in general, which induce collagen production. Concrete examples of such are salts of L-ascorbic acid, for example, inorganic salts of L-ascorbic acid such as sodium salt, potassium salt, calcium salt, magnesium salt, ammonium salt, alkyl ammonium salt, phosphate, and sulfate of L-ascorbic acid. Examples of other derivatives of L-ascorbic acid are precursors of L-ascorbic acid; saccharide derivatives of L-ascorbic acid such as L-ascorbic acid 2-glucoside and L-ascorbic acid 2-glycoside; organic or inorganic salts and esters of L-ascorbic acid such as acyl derivatives of L-ascorbic acid 2-glucoside, acyl derivative of L-ascorbic acid 2-glycoside, DL-α-tocopherol 2-L-ascorbic acid phosphoric diester, and L-ascorbic acid 2-phosphoric acid. In the present invention, one or more of these compounds can be used in an appropriate combination. Among the above L-ascorbic acids, saccharide derivatives of L-ascorbic acid including L-ascorbic acid 2-glucoside and L-ascorbic acid 2-glycoside are most preferably used in the present invention because of their advantageous ability of inducing collagen production, as well as their safeness and stability.

In the case of applying the agent for enhancing collagen production of the present invention to animals capable of producing L-ascorbic acid, L-glono-γ-lactone, i.e., a precursor of L-ascorbic acid, as L-ascorbic acids, can be used alone or in combination with L-ascorbic acids.

Varying depending on the form, dose, and administration route/frequency/period, the amount of L-ascorbic acids to be incorporated into the agent for enhancing collagen production of the present invention, usually, L-ascorbic acids are used in an amount of at least 0.01% (w/w), preferably, in the range of 0.1 to 99% (w/w), more preferably, 1 to 99% (w/w), and most preferably, 5 to 99% (w/w) in terms of the weight of L-ascorbic acid against the total weight of the composition. In the case that the amount of L-ascorbic acids to be incorporated into the agent is below the above lower limit, the desired function and effect may not be expected. The upper limit of L-ascorbic acids to be incorporated into the agent should be determined, considering its economical benefit and a possible side effect induced by an excessive intake by living bodies.

The term "fatty acids" as referred to as in the present invention means fatty acids and derivatives thereof in general, which exert the action of enhancing collagen production when used in combination with L-ascorbic acids. Concrete examples of such are straight or branched saturated or unsaturated fatty acids with carbon atom numbers of at least three, and derivatives thereof; preferably, straight or branched saturated or unsaturated fatty acids with carbon atom numbers of at least ten, and derivatives thereof; more preferably, straight or branched saturated or unsaturated fatty acids with carbon atom numbers of between 10 and 18, and derivatives thereof. Among the above fatty acids, 10-HDA, 10-hydroxydecanoic acid, decanoic acid, 2-decenoic acid, sebacic acid, and derivatives thereof are most preferably used. These fatty acids also enhance the production of transforming growth factor (hereinafter called "TGF-β") in fibroblasts in the presence of L-ascorbic acids. Since TGF-β is known to be produced by keratinocytes existing in intraepidermal tissues of the skin, it is suggested that fatty acids correlate to the mechanism of TGF-β production by keratinocytes.

The fatty acids used in the present invention should not be restricted to highly-purified ones and may include those with a relatively low purity as long as they do not spoil the desired object of the present invention. However, in the present invention, preferably used are purified fatty acids having a purity of at least 50%, preferably, at least 80%, more preferably, at least 90% and a lesser amount of impurities, which are obtainable either by extracting from natural products or by chemical synthesis. Concrete examples of such include fatty acids commercialized as reagents. As a method for preparing the fatty acids usable in the present invention from natural substances such as a raw royal jelly, the following method can be mentioned to collect fatty acids alone or others containing the same; a method containing the steps of suspending raw royal jelly in an aqueous solvent such as water, allowing the resulting suspension to stand or to centrifuge, resulting in separation into a supernatant fraction and a precipitate fraction; dissolving the precipitate by adding one or more alkaline agents selected from sodium hydroxide, potassium hydroxide, ammonia, sodium carbonate, sodium hydrogen carbonate, potassium carbonate, and ammonium carbonate; neutralizing the resulting solution with one or more acid agents selected from hydrochloric acid, nitric acid, sulfuric acid, lactic acid, and carnitine chloride; and subjecting the resulting solution to a means such as membrane separation and gel filtration to collect fatty acids alone or others containing the same. Usually, the above sequential procedures can be feasible at ambient temperature, and if necessary they can be carried out at temperatures in the range of 0 to 70°C while heating or cooling. Such a method for separating fatty acids is quite advantageous as the one for separating such fatty acids from raw royal jelly easily and effectively.

The agent for enhancing collagen production of the present invention is a composition containing the above-mentioned L-ascorbic acids and fatty acids as effective ingredients, where the fatty acids effectively enhance the action of inducing the collagen production by L-ascorbic acid, and therefore it can be provided in a variety of forms such as from those in the form of an agent for enhancing collagen production containing L-ascorbic acid in a relatively small amount to the extent that the action of enhancing collagen production could not be found when L-ascorbic acids are used alone to those in the form of an agent for enhancing collagen production, which exert a relatively high level of the action of enhancing collagen production to the extent that could not be exerted when L-ascorbic acids are used alone. In the agent for enhancing collagen production of the present invention, a preferable composition ratio of L-ascorbic acids and fatty acids is usually at least 0.0001 part by weight of fatty acids, preferably, 0.01 to 1 part by weight, and more preferably, 0.1 to 1 part by weight against one part by weight of L-ascorbic acids, in terms of the weight of L-ascorbic acid.

In the agent for enhancing collagen production of the present invention, the desired function and effect could not be expected when the composition ratio of fatty acids against L-ascorbic acids is below the above lower limit. While there exists no upper limit of the amount of fatty acids to be incorporated into L-ascorbic acids, it should preferably be provided, considering economics and affects on living bodies when administered in an excessive amount. The composition ratio of L-ascorbic acids and fatty acids as effective ingredients in the agent of the present invention is usually preferably set to a relatively low level, considering the form and the administration dose/frequency/period of the agent, as well as gender, age, body weight, and health conditions of living bodies such as animals including humans to be administered with the agent.

The action of enhancing collagen production exerted by the combination use of L-ascorbic acids and fatty acids can be examined using the assay for collagen production using human fibroblasts shown in the latter described Experiment 2.

The agent for enhancing collagen production of the present invention means a composition in general, which comprises substantially L-ascorbic acids and fatty acids and optionally one more other ingredients selected from materials for food products, health foods, foods for special use, cosmetics, pharmaceuticals, quasi-drugs (medicated cosmetics), feeds, baits, and pet foods. Examples of such other ingredients are antioxidants, viscosity-imparting agents, stabilizers, excipients, fillers, pH-controlling agents, sour agents, extracts, saccharides, sugar alcohols, amino acids, vitamins other than L-ascorbic acid (for example, vitamin B1, vitamin B2, vitamin B6, vitamin E, and vitamin P including rutin, naringin, and hesperidin), water, alcohols, amylaceous substances, proteins, fibers, lipids, minerals, flavors, colors, sweeteners, seasonings, spices, preservatives, emulsifiers, and surfactants, as well as glycosaminoglycans such as salts of chondroitin, chondroitin sulfate, dermatan sulfate, heparin, heparan sulfate, keratan sulfate, hyaluronic acid, and aluronic acid.

Among the above other ingredients, antioxidants are incorporated in the agent for enhancing collagen production of the present invention to inhibit the oxidative degradation of its effective ingredients. Examples of such antioxidants are flavonoids and polyphenols. The composition ratio of such antioxidants incorporated into the agent should not be specifically restricted, however, they are preferably used in a usual amount or in a lesser amount with respect to the composition ratios generally used in the field of food products.

Among the other ingredients, examples of saccharides include those which are glucose, fructose, lactose, trehalose, maltose, sucrose, lactosucrose, and starch hydrolyzates; other saccharides such as α-cyclodextrin, β-cyclodextrin, γ-cyclodextrin, and cyclic tetrasaccharide with a structure of cyclo{→6)-α-D-glucopyranosyl-(1→3)-α-D-glucopyranosyl-(1→6)-α-D-glucopyranosyl-(1→3)-α-D-glucopyranosyl-(1→}; sugar alcohols such as erythritol, mannitol, sorbitol, xylitol, maltitol, and hydrogenated starch hydrolyzates; sweeteners with a high sweetening power such as aspartame, stevioside extract, sucralose, acesulfam K, and saccharin; polysaccharides and natural gums such as pullulan, elsinan, carrageenan, xanthan gum, guar gum, gellan gum, and tamarind gum; and synthetic viscosity-imparting agents such as carboxymethyl cellulose. These saccharides can be advantageously used to sweeten, solidify, or stabilize the agent for enhancing collagen production of the present invention, as well as improving its taste.

Among the above other ingredients, examples of extracts are those which are prepared from buds of plants of the genus *Fagus crenata* of the family Fagaceae as disclosed in Japanese Patent Kokai No. 203,952/98; and other extracts such as ginkgo extract, pine extract, bamboo extract, Japanese apricot extract, paffia extract, beefsteak plant extract, indigo extract, and oyster extracts, which contain ingredients having an action of enhancing collagen production.

The form of the agent for enhancing collagen production of the present invention thus obtained can be illustrated with liquids, syrups, pastes, sheets, powders, granules, solids, pellets, capsules, etc.

When orally or parenterally (e.g., percutaneously) administering the agent for enhancing collagen production to mammals including humans, the effective ingredients of the agent are absorbed by the bodies to enhance the collagen production in fibroblasts present in the dermis and organs. By administering the agent to living bodies everyday or every other day, the collagen production *in vivo* can be maintained and the skin conditions can be effectively maintained. In addition, the agent treats the skin aging relating to aging and enhances the collagen production in the skin tissues damaged by external factors including ultraviolet ray, resulting in recovering the damaged skin to normal conditions. As a result, such skin will be imparted with resilience and moisture, resulting in improving or even diminishing wrinkles such as fine wrinkles. The agent can be also useful in preventing wrinkles such as fine wrinkles.

Varying depending on the kind, health condition, age, and gender (male or female) of animals, such as pet animals, domestic animals, poultry, and fish, including humans, the administration dose of L-ascorbic acids contained in the agent in terms of the weight of L-ascorbic acid is usually 0.1 to 500 mg/kg weight, preferably, 1 to 500 mg/kg weight, and more preferably, 1 to 250 mg/kg weight, when the agent is orally administered. The administration dose of fatty acids contained in the agent is at least 0.0001 part by weight, preferably, 0.01 to 1 part by weight, and more preferably, 0.1 to 1 part by weight to one part by weight of L-ascorbic acids in terms of the weight of L-ascorbic acid. The administration frequency of the agent is one to several times a day everyday or at an interval of one or more days, depending on the level of function and effect of the agent and the conditions of living bodies.

Varying depending on the kind, health condition, age, and gender (male or female) of animals, such as pet animals, domestic animals, poultry, and fish, including humans, the administration dose of L-ascorbic acids contained in the agent in terms of the weight of L-ascorbic acid is usually 0.1 to 500 mg/kg weight, preferably, 1 to 500 mg/kg weight, and more preferably, 1 to 250 mg/kg weight, when the agent is parenterally administered. The administration dose of fatty acids contained in the agent is at least 0.0001 part by weight, preferably, 0.01 to 1 part by weight, and more preferably, 0.1 to 1 part by weight of fatty acids to one part by weight of L-ascorbic acids in terms of the weight of L-ascorbic acid. The administration frequency of the agent is one to several times a day everyday or at an interval of one or more days, depending on the level of function and effect of the agent and the conditions of living bodies.

To prepare compositions containing the agent of the present invention, they are prepared by mixing adequate amounts of L-ascorbic acids and fatty acids and optionally an adequate amount of the already mentioned one or more ingredients selected from materials for food products, health foods, foods for special use, cosmetics, pharmaceuticals, quasi-drugs, feeds, baits, and pet foods; and processing the resulting mixtures into the desired forms in a usual manner to obtain different forms of compositions to meet the use of the desired agent. As long as L-ascorbic acids and fatty acids can be mixed in a prescribed ratio, the order and the timing of adding other ingredients are not specifically restricted. To prepare the agent of the present invention in a solid form, compositions incorporated with effective ingredients to be added to the agent and other appropriate ingredients are applied to conventional drying methods such as drying under a reduced pressure, drying *in vacuo,* and drying with hot air. Examples of such drying methods are, for example, in the case of using anhydrous α,α-trehalose as disclosed in Japanese Patent Kokai No. 170,221/94, a solid agent for enhancing collagen production can be easily obtained without employing a drying step by heating. Concrete examples of such are methods which comprise adding at least an equal amount of anhydrous saccharides such as anhydrous a,a-trehalose to the total amount of L-ascorbic acids and fatty acids, mixing the mixture, and usually allowing the resulting mixture to stand or stir at normal temperature or lower to form a solid. Since such a method does not necessarily require heating, it can advantageously avoid inactivating thermolabile L-ascorbic acids by heating. In addition to the above anhydrous α,α-trehalose, one or more of anhydrous α,β-trehalose, anhydrous glucose, and anhydrous maltose can be used in an appropriate combination.

The agent of the present invention in a solid form thus obtained can be optionally further pulverized, granulated or tabletted into a powder, granular, tablet, or pellet.

Examples of food products, health foods, and foods for special use incorporated with the agent of the present invention include ice milks such as an ice cream, ice candy, chou ice cream, and sherbet; syrups such as "*korimitsu*" (a sugar syrup for shaved ice); spreads and pastes such as a butter cream, custard cream, flour paste, peanut paste, and fruit paste; Western cakes such as a chocolate, jelly, mousse, candy, gummy jelly, caramel, chewing gum, pudding, Yorkshir pudding, chou, sponge cake, and pao *de Castella;* processed fruits and vegetables such as a jam, marmalade, "*syrup-zuke*" (fruit pickles), and "*toka*" (conserves); "*wagashi*" (Japanese cakes) such as "*manju*" (a bun with a bean-jam), "uiro" (a sweet rice jelly), "*an*" (a bean jam), "*yokan*" (a sweet jelly of beans), "*mizu-yokan*" (a soft adzuki-bean jelly), *pao* de *Castella,* "*amedama*" (a Japanese toffee), and rice confectionery including baked confectionery; seasonings such as a soy sauce, powdered soy sauce, *miso,* powdered *miso,* mayonnaise, dressing, vinegar, "*sanbai-zu*" (a sauce of sugar, soy sauce and vinegar), table sugar, and coffee sugar; alcohols such as a synthetic *sake,* fermented liquor, low malt beer, distilled spirit, wine, and liqueur; teas and beverages such as a juice, mineral beverage, carbonated beverage, lactic acid beverage, beverage with lactic acid bacteria, sports drink, tonic drink, green tea, black tea, and oolong tea; soft drinks such as coffee and cocoa; vitamins; minerals; proteins; medicinal carrot, Japanese apricot extract; paine extract; bamboo extract; ginkgo extract; mushroom extracts; royal jelly; propolis; herbs; low caloric foods; foods with reduced salt (sodium chloride); low-fat foods; natural foods; organic foods; and deep sea water.

Examples of cosmetics and quasi-drugs incorporated with the agent of the present invention are, for example, fundamental cosmetics such as an external skin lotion, cream, jelly, moose, deodorant, milky lotion, solid, powder, tooth paste, mouth-smell-inhibitory agent, mouth wash, soap, hand soap, bath salt, body shampoo, shampoo, rinse, pack, face mask, eye mask, and perfume; cosmetics for washing; cosmetics for bath; packs; oral cosmetics; cosmetics for sunburn or sunburn preventive; anti-perspirants; make-up cosmetics; and hair cosmetics such as a hair restorer, agent for hair growth, hair tonic, and styling spritz.

Examples of pharmaceuticals incorporated with the agent of the present invention are, for example, external skin agents and internal medicines such as a liquid, powder, granule, tablet, ointment, pellet, sheet, pap, and wet pack.

Examples of feeds, baits, and pet foods incorporated with the agent of the present invention are, for example, those which are obtained by appropriately incorporating the agent into conventional materials for feeds, baits, and pet foods.

Further, the agent of the present invention can be used by adding to groceries such as a kitchen detergent, sponge brush, hair band, and wrist band; feminine protection products such as a napkin; trousers; skirts; hats/caps; socks; stockings; handkerchiefs; towels; bath towels; gloves; bandages; corsets; shoes; and slippers by soaking, applying, spraying or adhering.

To produce the above-mentioned compositions, the agent of the present invention can be incorporated therein at any appropriate time before completion of their processings. Usually, when the compositions are processed through a heating step, the agent should preferably be added to the contents after a heating step to prevent heat inactivation of L-ascorbic acids.

The composition ratio of the agent of the present invention in the above compositions is usually at least 0.001% (w/w), preferably, 0.01 to 99% (w/w), and more preferably, 0.05 to 50% (w/w) to the weight of a final product.

By applying the agent of the present invention or various compositions incorporated therewith to living bodies, both of the L-ascorbic acids and fatty acids contained in the agent act on fibroblasts present in the living bodies' skin to enhance their collagen production. As a result, the following can be attained; reinforcement of the skin, enhancement of biological defensibility of the skin, prevention of the skin aging accompanied by aging, and normalization of the skin damaged by factors including ultraviolet ray. Further, application of the agent of the present invention to living bodies may be also expected to strengthen the tissues of internal organs and blood vessels.

Since the agent of the present invention imparts a useful function and effect on the skin as described above, it has an action of regenerating, growing or restoring hair. Accordingly, applying to pet animals, domestic animals, and poultry such as dogs, cats, horses, sheep, and birds with body fur or feather, the agent of the present invention will be expected to activate their skin and skin tissues, promote the regeneration of fur or feather on their bodies, and improve their fur or feather. In the case of applying to the skin such as human scalp, the agent of the present invention will be also expected to act on hair regeneration, growth, and restoration. Further, when the agent of the present invention and/or compositions containing the same are directly introduced into living bodies through their skin, the effective ingredients of the agent can be effectively introduced into the living bodies by using, for example, an apparatus for iontophoresis disclosed in International Patent Publication No. WO 01/60,388 applied for by the same applicant as the present invention.

The present invention will be explained in detail with reference to the following Experiments and Examples:

### Experiment 1

### <Separation and identification of ingredients, capable of enhancing collagen production, derived from royal jelly>

At ambient temperature, one gram of a royal jelly from Brazil was suspended in 10 ml of refined water and centrifuged at 3,000 rpm for 10 min to separate into a supernatant fraction and a precipitate fraction. To the precipitate fraction was added one milliliter of 1N-NaOH aqueous solution to dissolve solids in the fraction, followed by neutralizing the resulting solution with 1N-HCl aqueous solution, adding purified water to give a total volume of 10 ml, and fractionating the resulting solution using a UF-membrane with a molecular cut-off of 5,000 daltons into a high molecular fraction and a low molecular fraction. By applying the later described collagen assay (called "collagen assay" hereinafter) to each of the fractions, the presence or the absence of the action of enhancing collagen production in the fractions was examined. As a result, the action of enhancing collagen production was found to be highest in the low molecular fraction of the precipitate fraction (called "Fraction A"). Based on this finding, the present inventors tried to separate and identify ingredients for enhancing collagen production contained in Fraction A. Using C18 reverse-phase high performance liquid chromatography (called "HPLC" hereinafter), where employed was a column having an inner diameter of 4.6 mm, a column length of 250 mm, and packed with a gel made of silica gel to which octadecyl group bound (produced by Grace Vydac CA, USA), Fraction A was fractionated into a plurality of fractions which were then assayed with the collagen assay to identify and collect fractions with activity of enhancing collagen production. The conditions for elution were conducted in such a manner of sequentially feeding to the column, at an elution rate of 0.5 ml/min, 0.05% (w/v) aqueous trifluoroacetate solution for 15 min, 0.05% (w/v) aqueous trifluoroacetate solution containing 20% (w/v) acetonitrile for 5 min, and then a linear gradient increasing from 20% (w/v) to 80% (w/v) in 0.05% (w/v) aqueous trifluoroacetate solution for 60 min, while monitoring the absorption peak for eluted fractions at a wavelength of 210 nm, and collecting a fraction with a high absorption peak (Fraction B). The reason for monitoring the absorption peak at a wavelength of 210 nm was that, upon preliminary experiment, the ingredient with an action of enhancing collagen production contained in Fraction A was revealed to have the absorption wavelength. Upon re-chromatography of Fraction B on C18 reverse-phase HPLC conducted under the same conditions as used in the above, the ingredient with an action of enhancing collagen production (called "Ingredient X", hereinafter) was eluted at a position of 43 to 44 min. Ingredient X was examined for its maximum absorption peak and revealed to have a wavelength of 215 nm.

The present inventors estimated Ingredient X to be 10-hydroxy-2-decenoic acid (10-HDA) as a candidate based on the fact that Fraction A contained a relatively large amount of fatty acids upon HPLC analysis and 70% of the fatty acids was 10-HDA. Stating from the result, this estimation was correct. To clarify the identity of Ingredient X and 10-HDA, a commercialized high-purity reagent of 10-HDA (produced by Azwell Co., Ltd., Osaka, Japan) was subjected to C18 reverse-phase HPLC under the same conditions as used in the above and eluted at a position of 43.8 min. The elution position coincided with that of Ingredient X. 10-HDA had a maximum absorption at a wavelength of 215 nm, which coincided with that of Ingredient X. Further, Ingredient X and 10-HDA were subjected to thin-layer silica gel chromatography (5 x 15 cm) to compare their mobilities, revealing that they completely coincided with each other. To examine the identity between Ingredient X and 10-HDA, they were examined for their action of enhancing collagen production by the collagen assay, revealing that 10-HDA did not show an action of inducing collagen production when used alone but exhibited an action of enhancing the collagen production by L-ascorbic acid when used in combination with L-ascorbic acid. This result coincided with that of Ingredient X.

Measurement and comparison of mass spectra of Ingredient X and 10-HDA on mass spectrometric analysis revealed that their molecular ion peaks (m/z) of 85[M-H]-completely coincided with each other.

Based on these results, Ingredients X, purified and separated from raw royal jelly, was identified as 10-HDA.

### <Collagen assay>

NHDF cells, a human fibroblast, were seeded in a 96-well microplate (commercialized by Becton Dickinson, NJ, USA) with Eagle's modified Dulbecco's medium (designated as "DMEM medium" hereinafter, commercialized by Nissui Pharmaceutical Co., Ltd., Tokyo, Japan)(pH 7.1 to 7.4), supplemented with 10% (v/v) fetal calf serum and an adequate amount of penicillin, to give a cell density of 2.5 x 10⁴ cells/well, and incubated at 37°C for 24 hours under a 5% (v/v) CO₂ condition. Thereafter, the supernatant was replaced with 200 µl of a DMEM medium supplemented with a sample having a prescribed concentration and/or 50 µM of L-ascorbic acid 2-glucoside (a product name of "AA2G" with a purity of 98.0% or over, produced by Hayashibara Biochemical Laboratories, Inc., Okayama, Japan), and further cultured for another five days. After completion of the culture, the collagen production level from cells in each well of the microplate was quantified by using "Sircol™ Soluble Collagen Assay Kit", commercialized by Biocolar Ltd., Northern Ireland.

### Experiment 2

### <Action of enhancing the collagen production by 10-HDA>

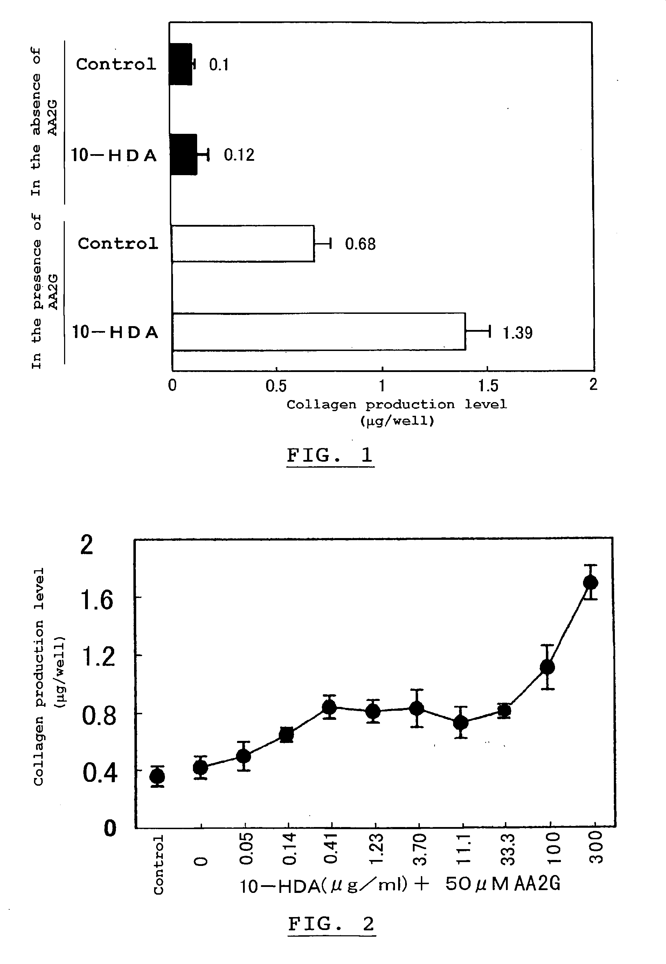

The action of enhancing the collagen production by 10-HDA was examined by the collagen assay shown in Experiment 1. In the collagen assay, 150 µg/ml of 10-HDA and/or a DMEM medium containing 50 µM of the same AA2G as used in Experiment 1 were used to examine the action of enhancing the collagen production by 10-HDA. As a control, there were provided two systems with 50 µM of AA2G or a DMEM medium free of AA2G, both of which were free of sample. The results are in FIG. 1.

As evident from the results in FIG. 1, 10-HDA did not show the action of enhancing collagen production in the absence of AA2G. From the result, it can be judged that 10-HDA *per* se has no action of enhancing collagen production. While 10-HDA showed a distinct action of enhancing collagen production when used in combination with AA2G. Based on these results, it was concluded that 10-HDA exerts an action of enhancing collagen production when used in combination with AA2G.

The relationship between the concentration of 10-HDA and the action of enhancing collagen production was also studied. In the collagen assay, using a DMEM medium containing 0 to 300 µg/ml of 10-HDA as a sample and 50 µM of AA2G, the action of enhancing the collagen production by 10-HDA was examined. As a control, a DMEM medium containing 50 µM of AA2G free of sample was provided. The results are in FIG. 2.

As evident from FIG. 2, 10-HDA showed a concentration-dependent action of enhancing collagen production in the presence of AA2G.

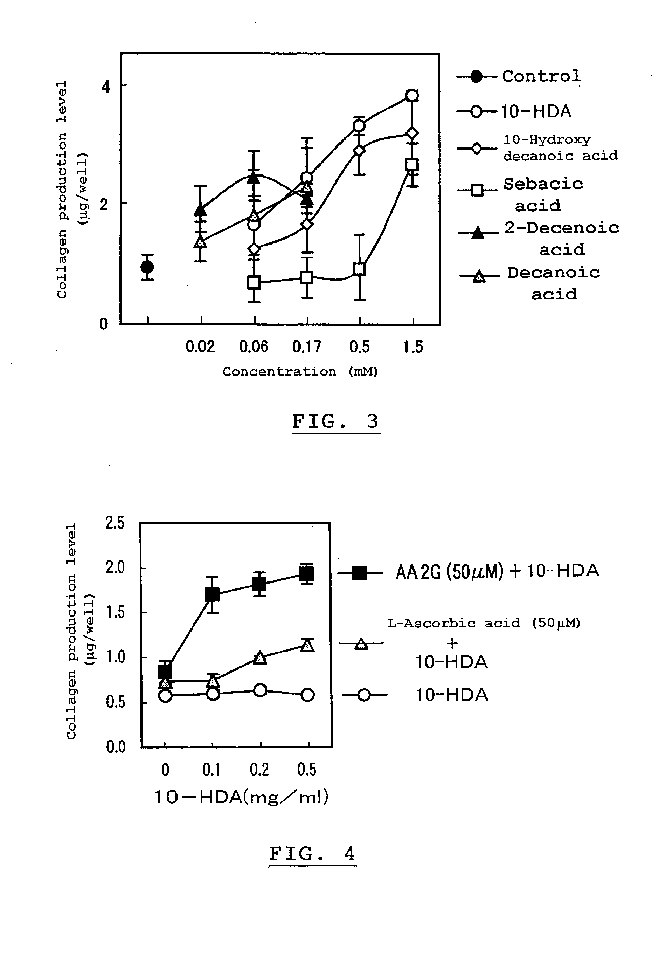

Similarly as in the above except for using 10-hydroxydecanoic acid (10-H decanoic acid), decanoic acid, 2-decenoic acid, and sebacic acid as fatty acids in place of 10-HDA, the actions of enhancing the collagen production by the above fatty acids were examined; using as a sample a DMEM medium containing 50 µM of AA2G and 0.02, 0.06, 0.17, 0.5 or 1.5 mM of any one of the above fatty acids, they were examined for their action of enhancing collagen production. The results are in FIG. 3.

As evident from FIG. 3, all of the fatty acids tested exerted a significant action of enhancing collagen production when used in combination with L-ascorbic acid. Among the fatty acids tested, 10-HDA had a significantly high action of enhancing collagen production, particularly, it gave a maximum at concentrations of 0.17 mM or over. It was also revealed that the levels of action of enhancing collagen production next to 10-HDA were in the order of 10-hydroxydecanoic acid, 2-decenoic acid, decanoic acid, and sebacic acid; and that the actions of these fatty acids were roughly in a concentration dependent manner.

### Experiment 3

### <Influence of 10-HDA on the action of enhancing collagen production in the presence of L-ascorbic acid or AA2G>

To examine the influence of 10-HDA on the action of enhancing the collagen production by L-ascorbic acid or AA2G, the following experiment was conducted: In the collagen assay shown in Experiment 1, the levels of collagen production in cell culture by L-ascorbic acid or 10-HDA were compared and analyzed using a DMEM medium containing as a sample 0, 0.1, 0.2 or 0.5 mg/ml of 10-HDA and 50 µM of L-ascorbic acid or AA2G as L-ascorbic acids in terms of L-ascorbic acid, and another DMEM medium containing as a sample 0, 0.1, 0.2 or 0.5 mg/ml of 10-HDA but free of L-ascorbic acids. The results are in FIG. 4.

As evident from the results in FIG. 4, 10-HDA showed an action of enhancing collagen production in a concentration dependent manner in the presence of L-ascorbic acid or AA2G. It was revealed that, when used with AA2G as one of L-ascorbic acids, the action of enhancing the collagen production by 10-HDA is more augmented by about two-times higher than that attained with L-ascorbic acid, revealing that the combination use of 10-HDA and AA2G is useful.

Although concrete data are not shown, saccharide derivatives of L-ascorbic acid tend to exert a more distinct action of enhancing collagen production when used in combination with fatty acids such as 10-HDA, comparing with those used in combination with derivatives of L-ascorbic acid such as salts, esters, and ethers of L-ascorbic acid. Among saccharide derivatives of L-ascorbic acid, AA2G exerts a more remarkable action of enhancing collagen production when used in combination with fatty acids.

### Experiment 4

### <Influence of fatty acids on the TGF-β production in human fibroblasts>

NHDF cells, a human fibroblast, commercialized by Kurabo Industries Ltd., Okayama, Japan, were seeded in 96-well microplates (commercialized by Becton Dickinson, NJ, USA) with a DMEM medium supplemented with 10% (v/v) fetal calf serum to give a cell density of 2.5 x 10⁴ cells/well, and incubated for 24 hours under a 5% (v/v) CO₂ condition. Thereafter, the supernatant in each microplate was removed, replaced with a fresh DMEM medium containing 0, 0.5 or 1.5 mM of 10-HDA and 0.2 µg/ml of AA2G, and cultured for 24 hours. After completion of the culture, the level of TGF-β in a supernatant of each well of the microplates was quantified by using "TGF-β1 E MAX IMMUNO ASSAY SYSTEM", commercialized by Promega Corp., WI, USA, and the data were statistically handled. The results are in Table 1.

**Table 1**

| 10-HDA concentration (mM) | TGF-β production level (µg/ml) |
|---|---|
| 0 | 26±46* |
| 0.5 | 292±68** |
| 1.5 | 378±74*** |

| | |
|---|---|
| Note: The symbols "*", "**", and "***" mean p<0.05, p<0.05 and p<0.01, respectively. | |

From the results in Table 1, it was revealed that, in the presence of AA2G, 10-HDA enhances the TGF-β production in human fibroblasts in a concentration dependent manner.

Although not showing concrete data, similarly as 10-HDA, fatty acids other than 10-HDA such as 10-hydroxydecanoic acid, decanoic acid, 2-decenoic acid, and sebacic acid also enhance TGF-β production.

The present invention will be explained in detail with reference to the following examples:

### Example 1

### <Agent for enhancing collagen production>

The following ingredients as indicated below were mixed to homogeneity in a prescribed composition ratio, and the resulting mixture was tabletted in a usual manner to obtain an agent for enhancing collagen production of the present invention, weighing 300 mg per tablet. The product can be preferably used as an agent for enhancing collagen production in animals including humans. Also the product has a function as an agent for enhancing TGF-β production.

| <Ingredients> | |
|---|---|
| L-Ascorbic acid 2-glucoside ("AA2G", a product name of and commercialized by Hayashibara Biochemical Laboratories, Inc., Okayama, Japan) | 20 parts by weight |
| 10-HDA | 0.1 part by weight |
| Pullulan | 5 parts by weight |
| Refined water | 5 parts by weight |

### Example 2

### <Agent for enhancing collagen production>

The following ingredients as indicated below were mixed to homogeneity in a prescribed composition ratio, and the resulting mixture was spread over a plastic plate, followed by drying overnight at 45°C and cutting into a 2-cm-square and 0.5 mm in thickness to obtain an agent for enhancing collagen production of the present invention. By using the product previously applied with water or a cosmetic lotion daily in such a manner of orally taking or placing on a part of the skin, it effectively enhances the collagen production in the skin, prevent or improve fine wrinkles, and impart resilience and moisture to the skin.

| <Ingredients> | |
|---|---|
| L-Ascorbic acid 2-glucoside ("AA2G", a product name of and commercialized by Hayashibara Biochemical Laboratories, Inc., Okayama, Japan) | 1 part by weight |
| 10-HDA | 0.01 part by weight |
| Anhydrous crystalline maltose ("FINETOSE", a product name of and commercialized by Hayashibara Shoji, Co., Ltd., Okayama, Japan) | 1 part by weight |
| Sucralose | 0.01 part by weight |
| Pullulan | 10 parts by weight |
| Refined water | 20 parts by weight |

### Example 3

### <Agent for enhancing collagen production>

The following ingredients as indicated below were mixed to homogeneity in a prescribed composition ratio, and the resulting mixture was membrane-filtered, injected into a 200-ml piston spraying container to obtain an agent for enhancing collagen production of the present invention. When applied by spraying to human or animal skin in an adequate amount, the agent enhances the collagen production in their skin and effectively improves their hair/fur/feather growth, regeneration, and condition. Also the product has a function as an agent for enhancing TGF-β production.

| <Ingredients> | |
|---|---|
| L-Ascorbic acid 2-glucoside ("AA2G", a product name of and commercialized by Hayashibara Biochemical Laboratories, Inc., Okayama, Japan) | 20 parts by weight |
| 10-HDA | 10 parts by weight |
| Refined water | 100 parts by weight |
| Preservative | 1 part by weight |

### Example 4

### <Health food>

The following ingredients as indicated below were mixed to homogeneity in a prescribed composition ratio, and the resulting mixture was in a usual manner dried overnight under a reduced pressure and at normal temperature, followed by pulverizing the resultant with a crasher to obtain a health food that functions as an agent for enhancing collagen production of the present invention. The product can be preferably used as an agent for enhancing collagen production for animals including humans. Also the product has a function as an agent for enhancing TGF-β production.

| <Ingredients> | |
|---|---|
| L-Ascorbic acid 2-glucoside ("AA2G", a product name of and commercialized by Hayashibara Biochemical Laboratories, Inc., Okayama, Japan) | 1 part by weight |
| Ingredient X or 10-HDA obtained by purification in Experiment 1 | 0.05 part by weight |
| Crystalline trehalose dihydrate ("TREHA®", a product name of and commercialized by Hayashibara Shoji, Co., Ltd., Okayama, Japan) | 2 parts by weight |

The product, which has a mild sweetness, an adequate acid taste, and a relatively long shelf-life and stability, is useful as a health food with an action of enhancing collagen production. Since the product can be easily taken alone, together with a solvent such as water, or after dissolving or suspending in such a solvent, it can be used as a composition for oral intake or intubation administration for animals including pet animals and domestic animals, as well as humans; and also used intact or after mixing with baits for fishery for cultivation such as fish, eels, shrimps, and crabs.

### Example 5

### <Health food>

The following ingredients as indicated below were mixed to homogeneity in a prescribed composition ratio, and the resulting mixture was dried *in vacuo* to obtain a health food that functions as an orally administrable powdery agent for enhancing collagen production with a moisture content of 5%. The product can be advantageously used as an agent for enhancing collagen production for humans, domestic animals, poultry, pet animals including insects, and aquarium fish. Also the product has a function as an agent for enhancing TGF-β production.

| <Ingredients> | |
|---|---|
| Sodium L-ascorbic acid | 1.5 parts by weight |
| L-Ascorbic acid 2-glucoside ("AA2G", a product name of and commercialized by Hayashibara Biochemical Laboratories, Inc., Okayama, Japan) | 1.0 part by weight |
| 10-HDA | 0.25 part by weight |
| Saccharide-transferred rutin ("αG RUTIN", a product name of and commercialized by Hayashibara Shoji, Co., Ltd., Okayama, Japan) | 1.0 part by weight |
| Anhydrous crystalline maltitol | 1.5 parts by weight |
| Trehalose | 0.2 part by weight |
| Refined water | 10.0 parts by weight |

### Example 6

### <Ice cream>

A mixture of 15 parts by weight of a fresh cream (about 46% (w/w) of fats and lipids), three parts by weight of a skimmed milk powder, 45 parts by weight of a whole milk, 10 parts by weight of trehalose, and one part by weight of pullulan was dissolved by heating, kept at 65°C for 30 min for sterilization, emulsified and dispersed with a homogenizer, promptly cooled to 4°C, and admixed with 0.5 part by weight of the agent for enhancing collagen production in Example 1, followed by mixing the mixture, injecting 100 g aliquots of which into appropriate containers, and freezing the resultant at -20°C to obtain an ice cream.

The product is an ice cream, which has an action of enhancing collagen production and an adequate sweetness and high-quality flavor and taste and which is capable of imparting moisture to the skin and preventing the aging of skin when daily taken.

### Example 7

### <Fruit jelly>

Among the following ingredients, sucrose, trehalose, gelatin and water were placed in an adequate container and dissolved by heating at 95°C, and then orange juice was added to the solution, followed by sterilizing the resulting mixture by heating at 80°C for 30 min, admixing with L-ascorbic acid 2-glucoside ("AA2G", a product name of and commercialized by Hayashibara Biochemical Laboratories, Inc., Okayama, Japan), and a royal jelly with pre-heating treatment, and cooling the resulting mixture to obtain a fruit jelly.

| <Ingredients> | |
|---|---|
| Sucrose | 10.0 parts by weight |
| Hydrous crystalline trehalose | 2.0 parts by weight |
| Gelatin | 2.5 parts by weight |
| Orange juice with 3% L-ascorbic acid | 32.0 parts by weight |
| Refined water | 45.0 parts by weight |
| L-Ascorbic acid 2-glucoside ("AA2G", a product name of and commercialized by Hayashibara Biochemical Laboratories, Inc., Okayama, Japan) | 1.0 part by weight |
| 10-Hydroxydecanoic acid | 0.01 part by weight |
| 10-HDA | 0.01 part by weight |

The product is a fruit jelly, which has an action of enhancing collagen production and an adequate sweetness and mild texture, maintains the skin in a healthy condition, prevents aging of the skin, and maintains/promotes the health and beauty.

### Example 8

### <Health beverage>

The following ingredients were mixed to homogeneity in a prescribed ratio, and 25 parts by weight of the mixture were dispersed and dissolved in 150 parts by weight of refined water. Two hundred grams aliquots of the resulting mixture were distributed into brown glass vials and sterilized by heating at 65°C for 30 min to obtain a health beverage.

| <Ingredients> | |
|---|---|
| Anhydrous crystalline maltose | 500 parts by weight |
| L-Ascorbic acid 2-glucoside ("AA2G", a product name of and commercialized by Hayashibara Biochemical Laboratories, Inc., Okayama, Japan) | 30 parts by weight |
| Sodium L-ascorbate | 6 parts by weight |
| 10-HDA | 2 parts by weight |
| 10-Hydroxydecanoic acid | 1 part by weight |
| Powdered egg | 190 parts by weight |
| Skimmed milk powder | 200 parts by weight |
| Sodium chloride | 4.4 parts by weight |
| Potassium chloride | 1.85 parts by weight |
| Magnesium sulfate | 4 parts by weight |
| Thiamine | 0.01 part by weight |
| Vitamin E acetate | 0.6 part by weight |
| Nicotinic acid amide | 0.04 part by weight |
| Saccharide-transferred hesperidin ("αG RUTIN", commercialized by Toyo Sugar Refining Co., Ltd., Tokyo, Japan,) | 0.02 part by weight |

The product can be advantageously used as a beverage for beauty and health, which has an action of enhancing collagen production, and it can be also advantageously used as a composition for intubation administration.

### Example 9

### <External skin cream>

To the ingredient 1 with the following prescribed composition was added the ingredient 2 with the following prescribed composition, followed by mixing the mixture while heating and stirring at 60°C. After cooling to ambient temperature, the resulting mixture was admixed with the ingredient 3 with the following prescribed composition, adjusted to pH 6.5 with potassium hydroxide and emulsified and dispersed by a homogenizer to produce an external skin cream having an action of enhancing collagen production.

| <Ingredient 1> | |
|---|---|
| Polyoxyethylene glyceryl monostearate | 2.0 parts by weight |
| Glyceryl monostearate, self-emulsifying | 5.0 parts by weight |
| Eicosanyl behenate | 1.0 part by weight |
| Liquid paraffin | 1.9 parts by weight |
| Trimethyrol trioctanoate | 10.0 parts by weight |

| <Ingredient 2> | |
|---|---|
| 1,3-Butylene glycol | 5.0 parts by weight |
| Sodium lactate solution | 10.0 parts by weight |
| Ginseng extract | 1.5 parts by weight |
| Methyl-para-oxybenzoate | 0.1 part by weight |
| Sodium hyaluronate | 0.1 part by weight |
| Chondroitin sulfate | 0.1 part by weight |
| Licorice extract | 0.5 part by weight |
| Refined water | 62.4 parts by weight |

| <Ingredients 3> | |
|---|---|
| L-ascorbic acid 2-glucoside ("AA2G", a product name of and commercialized by Hayashibara Biochemical Laboratories, Inc., Okayama, Japan) | 2 parts by weight |
| 10-HDA | 0.01 part by weight |
| Sebacic acid | 0.01 part by weight |

The product can be used as a basic cosmetic because of the following reasons; it enhances the production of collagen in the skin while inducing the TGF-β production by fibroblasts, maintains the fresh and youthful skin, imparts tautness and moisture to the skin, prevents pouching in the skin, and has a satisfactory moisture-imparting effect on the skin.

### Industrial Applicability

When orally or parenterally applied to animals including humans, the agent for enhancing collagen production of the present invention surely and stably enhances the collagen production in such living bodies' skin. By applying the agent to such animals including humans, it exerts a satisfactory function and effect; it prevents aging, promotes the growth of and maintains body fur/feather, and has usefulness in maintaining and promoting beauty and health.

Both of L-ascorbic acids and fatty acids as the effective ingredients in the agent of the present invention are ingredients which can be freely incorporated into food products, cosmetics, and pharmaceuticals without fear of causing side effects. Also, in the agent of the present invention, these effective ingredients can be incorporated precisely in a desired amount and therefore the quality of the agent can be quite easily controlled on an industrial scale production. Since the agent of the present invention does not contain costly royal jelly or the like, it has also the merit that it can be advantageously produced at a lesser cost in comparison with conventional agents for enhancing the collagen production, which contains L-ascorbic acids and royal jelly or the like.

Therefore, the agent for enhancing collagen production of the present invention can be used intact as such agent or used as compositions in various forms of food products, health foods, foods for special use, cosmetics, pharmaceuticals, quasi-drugs, feeds, baits, and pet foods by incorporating into one or more materials for the above compositions.

According to the method of separating fatty acids from raw royal jelly disclosed in this specification, the fatty acids usable as the effective ingredients in the agent for enhancing collagen production of the present invention, can be easily prepared on an industrial scale at a relatively low cost.

The present invention, having these distinct function and effect, is a significantly useful invention that greatly contributes to this art.

## Claims

1. An agent for enhancing collagen production, which comprises L-ascorbic acid and/or its derivative and a fatty acid or its derivative as effective ingredients.

2. The agent of claim 1, which contains said L-ascorbic acid and/or its derivative in an amount of 0.01% (w/w), in terms of the weight of L-ascorbic acid, to the total weight of said agent.

3. The agent of claim 1 or 2, which contains a fatty acid or its derivative in an amount of at least 0.0001 part by weight to one part by weight of said L-ascorbic acid and/or its derivative, in terms of the weight of L-ascorbic acid.

4. The agent of claim 1, 2 or 3, wherein said fatty acid or its derivative is derived from royal jelly.

5. The agent of any one of claims 1 to 4, wherein said fatty acid or its derivative is one or more members selected from the group consisting of 10-hydroxy-2-decenoic acid, 10-hydroxydecanoic acid, decanoic acid, 2-decenoic acid, and sebacic acid.

6. The agent of any one of claims 1 to 5, which further contains one or more other ingredients selected from materials for food products, foods for special use, cosmetics, pharmaceuticals, quasi-drugs, feeds, baits, and pet foods.

7. The agent of claim 6, wherein said other ingredient is one or more members selected from the group consisting of antioxidants, viscosity-imparting agents, stabilizers, excipients, fillers, pH-controlling agents, viscosity-imparting agents, sour agents, extracts, saccharides, glycosaminoglycans, sugar alcohols, amino acids, vitamins including L-ascorbic acid and derivatives thereof, water, alcohols, amylaceous substances, proteins, fibers, lipids, minerals, flavors, colors, sweeteners, seasonings, spices, preservatives, emulsifiers, and surfactants.

8. The agent of any one of claims 1 to 7, wherein said L-ascorbic acid derivative is one or more members selected from the group consisting of salts of L-ascorbic acid, L-ascorbic acid 2-glycoside, and L-ascorbic acid 2-glucoside.

9. The agent of claim 8, wherein said L-ascorbic acid 2-glycoside contains at least L-ascorbic acid 2-glucoside.

10. The agent of claim 7, wherein said glycosaminoglycan is one or more members selected from the group consisting of chondroitin, chondroitin sulfate, dermatan sulfate, heparin, heparan sulfate, keratan sulfate, hyaluronic acid, and aluronic acid.

11. A food product, health food, or food for special use, which comprises the agent of any one of claims 1 to 10.

12. A cosmetic, which comprises the agent of any one of claims 1 to 10.

13. A pharmaceutical or quasi-drug, which comprises the agent of any one of claims 1 to 10.

14. A feed, bait or pet food, which comprises the agent of any one of claims 1 to 10.

15. A process for producing the agent of any one of claims 1 to 10, which comprises a step of incorporating L-ascorbic acid and/or its derivative and a fatty acid or its derivative.

16. A method for separating a fatty acid or its derivative from a raw royal jelly, which comprises the steps of:
suspending said raw royal jelly in a solvent;
separating the resulting suspension into a supernatant fraction and a precipitate fraction by centrifugation and/or filtration;
dissolving the precipitate fraction with an alkaline agent;
neutralizing the resulting solution with an acid agent; and
separating the resulting neutralized solution with a membrane to collect said fatty acid or its derivative.
